(19) Europäisches Patentamt
European Patent Office
Office européen des brevets

(11) EP 4 785 942 A1

(12) EUROPEAN PATENT APPLICATION
published in accordance with Art. 153(4) EPC

(43) Date of publication:
**05.08.2026 Bulletin 2026/32**

(21) Application number: **24872388.4**

(22) Date of filing: **26.09.2024**

(51) International Patent Classification (IPC):
***A61K 31/496*** (2006.01) ***A61K 31/7052*** (2006.01)
***A61K 31/706*** (2006.01) ***A61K 45/00*** (2006.01)
***A61P 35/00*** (2006.01) ***A61P 35/02*** (2006.01)
***A61P 43/00*** (2006.01)

(52) Cooperative Patent Classification (CPC):
**A61K 31/496; A61K 31/7052; A61K 31/706; A61K 45/00; A61P 35/00; A61P 35/02; A61P 43/00**

(86) International application number:
**PCT/JP2024/034412**

(87) International publication number:
**WO 2025/070603 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 JP 2023165232**
**22.02.2024 JP 2024025883**

(71) Applicant: **Carna Biosciences, Inc.**
**Kobe-shi, Hyogo 650-0047 (JP)**

(72) Inventors:
- **ENDO, Hiroko**
  **Kobe-shi, Hyogo 650-0047 (JP)**
- **FURUICHI, Hatsuo**
  **Kobe-shi, Hyogo 650-0047 (JP)**
- **NISHIOKA, Yu**
  **Kobe-shi, Hyogo 650-0047 (JP)**
- **SAWA, Masaaki**
  **Kobe-shi, Hyogo 650-0047 (JP)**

(74) Representative: **Kador & Partner Part mbB**
**Corneliusstraße 15**
**80469 München (DE)**

# (54) NOVEL COMBINATION THERAPY FOR BLOOD CANCER

(57) The present invention provides a new means for treating a blood cancer by 1) a two-drug combination of a DNA methyltransferase inhibitor and a pharmaceutical composition containing a compound (I):

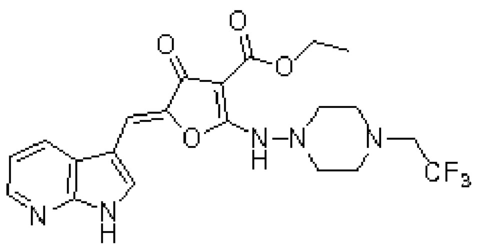

(I)

or a pharmaceutically acceptable salt or hydrate thereof as an active ingredient, and 2) a three-drug combination of a DNA methyltransferase inhibitor, a BCL-2 inhibitor, and a pharmaceutical composition containing a compound (I) or a pharmaceutically acceptable salt or hydrate thereof as an active ingredient.

[Figure 6A]

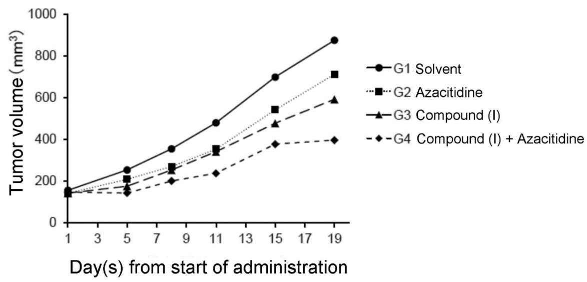


EP 4 785 942 A1

**Description**

TECHNICAL FIELD

The present invention relates to a novel means for treating a blood cancer. More specifically, the present invention relates to the provision of a new treatment means by a combination of a compound (I) or a pharmaceutically acceptable salt or hydrate thereof, and a DNA methyltransferase inhibitor, or further combining a BCL-2 inhibitor with the above combination of a compound (I) or a pharmaceutically acceptable salt or hydrate thereof and a DNA methyltransferase inhibitor.

BACKGROUND ART

Blood cancer is a general term for diseases in which blood cells become cancerous, and broadly classified into leukemia, malignant lymphoma, multiple myeloma, and others. In recent years, various treatment methods and anticancer drugs have been developed and are used to treat these blood cancers.

Myelodysplastic syndrome (MDS) is a disease where abnormalities occur in the hematopoietic stem cells within the bone marrow, thereby the production of normal blood is prevented. Approximately 30% of MDS patients are believed to progress to acute myeloid leukemia (AML). In recent years, abnormal DNA methylation, which induces the inactivation or the like of tumor suppressor genes, has been considered as one cause of cancer development and leukemic transformation.

DNA methyltransferase inhibitors have been developed to suppress this abnormal DNA methylation. DNA methyltransferase inhibitors, represented by azacitidine and decitabine, share a chemical structure similar to cytidine, and as a result, they are incorporated into a DNA within a living body, inhibit the DNA methyltransferase, and suppress the DNA methylation, thereby inducing the DNA damage and apoptosis, as well as exerting antitumor effects.

DNA methyltransferase inhibitors such as azacitidine and decitabine are already used as therapeutic agents for MDS and AML. However, a clinical issue arises when a patient shows no therapeutic response, or the medication loses its effectiveness during the treatment, which leads to drug resistance (Nonpatent Document 1).

BCL-2 (B-cell lymphoma 2) is a member of BCL-2 family that regulates cell death and is known to inhibit apoptosis in certain blood cancers. To date, several BCL-2 inhibitors including venetoclax have been reported. These BCL-2 inhibitors are known to selectively bind to BCL-2, thereby inhibiting the function of BCL-2, powerfully inducing apoptosis as each monotherapy or in combination with other therapeutic agent(s), and demonstrating the antitumor effects (Nonpatent Document 2). Venetoclax is approved as a combination therapy with a DNA methyltransferase inhibitor or the like such as azacitidine and decitabine for treating AML patients. However, even with this combination therapy, there are issues such as serious side effects making continued treatment difficult and insufficient therapeutic effects. Thus, the development of further new treatment methods is desired (Non-Patent Document 3).

CDC7 (cell division cycle 7) is a serine/threonine kinase and an essential enzyme for initiating a DNA replication during the cell cycle. CDC7 forms a complex with a cofactor such as Dbf4 (ASK) that activates its phosphorylation activity, thereby phosphorylating the substrate MCM (minichromosome maintenance) protein. This phosphorylation is thought to assemble Cdc45 and a DNA polymerase onto a DNA to form a MCM complex and initiate a DNA replication (Nonpatent Document 4). In recent years, CDC7 has gained attention as a target for anticancer agents. AS-0141, which selectively and potently inhibits CDC7, has been shown to exhibit antitumor activities against various cancer cell lines (Nonpatent Document 5).

AS-0141 is a compound represented by ethyl 5-[(1H-pyrrolo[2,3-b]pyridin-3-yl)methylene]-4-oxo-2-{[4-(2,2,2-trifluoroethyl)piperazinyl]amino}-4,5-dihydrofuran-3-carboxylate [compound (I)].

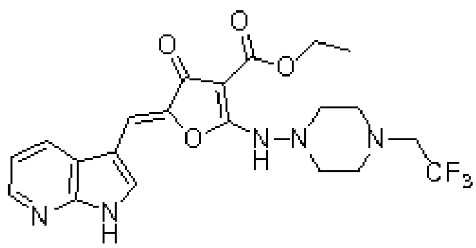

(I)

The compound (I) is disclosed in WO 2012/133802 pamphlet (Patent Document 1) and Nonpatent Document 5. Also, it is disclosed that a combination of the compound (I) and a M-phase promoter such as a Wee1 inhibitor enhances the

anticancer activities (Patent Document 2). WO 2018/84266 pamphlet discloses a method for treating a cancer comprising the compound (I) (Patent Document 3). WO 2020/68347 pamphlet discloses a treatment method which comprises administering SRA141 (AS-0141) in a combination therapy (Patent Document 4).

CITATION LIST

PATENT DOCUMENT

Patent Document 1: WO 2012/133802 pamphlet
Patent Document 2: WO 2015/115355 pamphlet
Patent Document 3: WO 2018/84266 pamphlet
Patent Document 4: WO 2020/68347 pamphlet

NON-PATENT DOCUMENT

Nonpatent Document 1: Zhao G, et al., Front Oncol. 2021 Sep 28;11:706030.
Nonpatent Document 2: Ashkenazi, A., et al., Nature Rev. Drug Discov., 2017, 16, 273-284.
Nonpatent Document 3: Estey EH., Am J Hematol. 2020 Nov; 95(11): 1368-1398.
Nonpatent Document 4: Sawa M, Masai H., Drug Des Devel Ther., 2008 Feb 6; 2: 255-264.
Nonpatent Document 5: Irie T, et al., J Med Chem. 2021 Oct 14;64(19): 14153-14164.

SUMMARY OF THE INVENTION

PROBLEMS TO BE SOLVED BY THE INVENTION

An object of the present invention is to provide a new method for treating a blood cancer patient, the method showing higher therapeutic effects and fewer side effects.

MEANS TO SOLVE PROBLEMS

A combination of a compound (I) or a pharmaceutically acceptable salt or hydrate thereof and a DNA methyltransferase inhibitor has not been reported at all, and a finding that a combination of a compound (I) or a pharmaceutically acceptable salt or hydrate thereof and a DNA methyltransferase inhibitor increases the anticancer effects against blood cancers has also not been reported at all. Further, a three-drug combination of a compound (I) or a pharmaceutically acceptable salt or hydrate thereof, a DNA methyltransferase inhibitor, and a BCL-2 inhibitor has not been reported at all, and a finding that said three-drug combination increases the anticancer effects against blood cancers has also not been known at all.

The present inventors have earnestly studied in order to solve the above problem. As a result, they have found that the problem of the present invention is solved by administering a combination of 1) a compound (I) or a pharmaceutically acceptable salt or hydrate thereof and 2) a DNA methyltransferase inhibitor, and also a combination of the combination of these 1) and 2), and additionally 3) a BCL-2 inhibitor, thereby achieving the synergistic effects, to complete the present invention.

More specifically, the present invention relates to the followings. In the present description, a compound (I) encompasses a pharmaceutically acceptable salt or hydrate thereof, unless otherwise specified. The same is also applied to azacitidine and decitabine.

(1) A pharmaceutical composition comprising a compound (I):

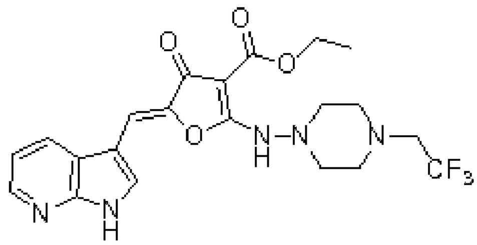

(I)

as an active ingredient, which is administered to a patient in combination with a DNA methyltransferase inhibitor for use in the treatment of a blood cancer.

(2) The pharmaceutical composition according to (1), which is administered to the patient further in combination with a BCL-2 inhibitor in addition to the DNA methyltransferase inhibitor for use in the treatment of the blood cancer.

(3) The pharmaceutical composition according to (1) or (2), wherein the blood cancer is myelodysplastic syndrome (MDS) and/or acute myeloid leukemia (AML).

(4) The pharmaceutical composition according to (1) or (2), wherein the blood cancer is myelodysplastic syndrome (MDS).

(5) The pharmaceutical composition according to (1) or (2), wherein the blood cancer is acute myeloid leukemia (AML).

(6) The pharmaceutical composition according to (1) or (2), wherein the DNA methyltransferase inhibitor is a pharmaceutical composition comprising azacitidine or decitabine as an active ingredient.

(7) The pharmaceutical composition according to (6), wherein the DNA methyltransferase inhibitor is a pharmaceutical composition comprising azacitidine as an active ingredient.

(8) The pharmaceutical composition according to (6), wherein the DNA methyltransferase inhibitor is a pharmaceutical composition comprising decitabine as an active ingredient.

(9) The pharmaceutical composition according to (7), wherein the pharmaceutical composition comprising azacitidine as an active ingredient, and the pharmaceutical composition comprising the compound (I) as an active ingredient are administered both simultaneously, or separately and sequentially.

(10) The pharmaceutical composition according to (8), wherein the pharmaceutical composition comprising decitabine as an active ingredient, and the pharmaceutical composition comprising the compound (I) as an active ingredient are administered both simultaneously, or separately and sequentially.

(11) The pharmaceutical composition according to (2), wherein the BCL-2 inhibitor is a pharmaceutical composition comprising venetoclax as an active ingredient.

(12) The pharmaceutical composition according to any one of (9) to (11), wherein the blood cancer is myelodysplastic syndrome (MDS) and/or acute myeloid leukemia (AML).

(13) The pharmaceutical composition according to (12), wherein the blood cancer is myelodysplastic syndrome (MDS).

(14) The pharmaceutical composition according to (12), wherein the blood cancer is acute myeloid leukemia (AML).

(15) A kit comprising a pharmaceutical composition comprising the DNA methyltransferase inhibitor and the compound (I), each as an active ingredient, for use in the combined administration as defined in (1).

(16) A kit comprising a pharmaceutical composition comprising the DNA methyltransferase inhibitor, the BCL-2 inhibitor, and the compound (I), each as an active ingredient, for use in the combined administration as defined in (2).

(17) A method for treating a blood cancer, the method comprising administering a pharmaceutical composition comprising a compound (I) as an active ingredient to a patient, in combination with a DNA methyltransferase inhibitor.

(18) A method for treating a blood cancer, the method comprising administering a pharmaceutical composition comprising a compound (I) as an active ingredient to a patient, in combination with a DNA methyltransferase inhibitor and a BCL-2 inhibitor.

(19) The method according to (17) or (18), wherein the blood cancer is myelodysplastic syndrome (MDS) and/or acute myeloid leukemia (AML).

(20) The method according to (17) or (18), wherein the DNA methyltransferase inhibitor is a pharmaceutical composition comprising azacitidine or decitabine as an active ingredient.

(21) The method according to (18), wherein the BCL-2 inhibitor is a pharmaceutical composition comprising venetoclax as an active ingredient.

EFFECT OF INVENTION

A combination of the compound (I) or a pharmaceutically acceptable salt or hydrate thereof and a DNA methyltransferase inhibitor can induce cell death more efficiently than when each of the compound (I) or the DNA methyltransferase inhibitor is used alone. The CDC7 inhibitor can cause genomic instability in cancer cells, thereby inducing a DNA damage and apoptosis. On the other hand, the DNA methyltransferase inhibitor suppresses abnormal DNA methylation in cancer cells, thereby activating tumor suppressor genes and inducing DNA damages and apoptosis. Therefore, a combination of these compound (I) and DNA methyltransferase inhibitor is expected to exhibit enhanced anticancer activities, thereby potentially achieving high therapeutic effects against various blood cancers.

Furthermore, by a three-drug combination of the compound (I) or a pharmaceutically acceptable salt or hydrate thereof, the DNA methyltransferase inhibitor, and further the BCL-2 inhibitor, further synergistic effects can be expected, thereby achieving higher therapeutic effects against various blood cancers.

BRIEF DESCRIPTION OF DRAWINGS

[Figure 1] Figure 1 is a figure of the cell proliferation inhibition rate showing the interaction by a combination of a DNA methyltransferase inhibitor (azacitidine) and the compound (I) against a human acute monocytic leukemia cell line (THP-1).

[Figure 2A] Figure 2A is a figure showing effects of a combination of a DNA methyltransferase inhibitor (azacitidine) and the compound (I) against THP-1 cells as changes in the expression levels of a DNA damage indicator, γH2AX, and an apoptosis indicator, cleaved caspase 3.

[Figure 2B] Figure 2B is a figure showing effects of a combination of a DNA methyltransferase inhibitor (decitabine) and the compound (I) against MV-4-11 cells as changes in the expression levels of a DNA damage indicator, γH2AX, and an apoptosis indicator, cleaved caspase 3.

[Figure 3] Figure 3 is a figure of apoptosis induction by a combination of a DNA methyltransferase inhibitor (azacitidine) and the compound (I) against THP-1 cells.

[Figure 4A] Figure 4A is a figure of isobologram (50% inhibition) showing the interaction by a combination of a DNA methyltransferase inhibitor (azacitidine) and the compound (I) against THP-1 cells.

[Figure 4B] Figure 4B is a figure of isobologram (50% inhibition) showing the interaction by a combination of a DNA methyltransferase inhibitor (azacitidine) and the compound (I) against a human acute myeloid leukemia cell line (MV-4-11).

[Figure 4C] Figure 4C is a figure of isobologram (50% inhibition) showing the interaction by a combination of a DNA methyltransferase inhibitor (azacitidine) and the compound (I) against a human acute monocytic leukemia cell line (MOLM-14).

[Figure 4D] Figure 4D is a figure of isobologram (50% inhibition) showing the interaction by a combination of a DNA methyltransferase inhibitor (azacitidine) and the compound (I) against a human erythroleukemia cell line (TF-1).

[Figure 4E] Figure 4E is a figure of isobologram (50% inhibition) showing the interaction by a combination of a DNA methyltransferase inhibitor (decitabine) and the compound (I) against a human acute myeloid leukemia cell line (MV-4-11).

[Figure 4F] Figure 4F is a figure of isobologram (50% inhibition) showing the interaction by a combination of a DNA methyltransferase inhibitor (decitabine) and the compound (I) against a human acute monocytic leukemia cell line (MOLM-14).

[Figure 4G] Figure 4G is a figure of isobologram (50% inhibition) showing the interaction by a combination of a DNA methyltransferase inhibitor (decitabine) and the compound (I) against a human erythroleukemia cell line (TF-1).

[Figure 5] Figure 5 is a Fa-CI plot showing the interaction by a combination of a DNA methyltransferase inhibitor (azacitidine), a BCL-2 inhibitor (venetoclax), and the compound (I) against a human acute myeloid leukemia cell line (MV-4-11).

[Figure 6A] Figure 6A shows the tumor growth suppression by a combination of the compound (I) and a DNA methyltransferase inhibitor (azacitidine) in a mouse subcutaneous transplantation model of a human acute myeloid leukemia cell line (MV-4-11).

[Figure 6B] Figure 6B shows the tumor growth suppression by a combination of the compound (I) and a DNA methyltransferase inhibitor (decitabine) in a mouse subcutaneous transplantation model of a human acute myeloid leukemia cell line (MV-4-11).

[Figure 7A] Figure 7A shows the tumor growth suppression by a combination of the compound (I), a DNA methyltransferase inhibitor (azacitidine), and a BCL-2 inhibitor (venetoclax) in a mouse subcutaneous transplantation model of a human acute myeloid leukemia cell line (MV-4-11).

[Figure 7B] Figure 7B shows the tumor growth suppression by a combination of the compound (I), a DNA methyltransferase inhibitor (decitabine), and a BCL-2 inhibitor (venetoclax) in a mouse subcutaneous transplantation model of a human acute myeloid leukemia cell line (MV-4-11).

MODE FOR CARRYING OUT THE INVENTION

(1) DNA methyltransferase inhibitor

In the present invention, a DNA methyltransferase inhibitor refers to a pharmaceutical composition comprising a drug which inhibits the DNA methyltransferase activities as an active ingredient, and encompasses a pharmaceutical composition comprising a drug which inhibits physiological functions of the DNA methyltransferase such as a low molecular weight compound, a polypeptide, a protein, a nucleic acid (siRNA, miRNA, aptamer, or the like), and another high molecular weight compound. Further, it also encompasses a drug which is activated in a living body to inhibit the physiological functions of the DNA methyltransferase, i.e., a prodrug of the DNA methyltransferase inhibitor.

Examples of the drug which inhibits DNA methyltransferase activities include azacitidine and decitabine having the following structures, as well as their pharmaceutically acceptable salts, hydrates, and prodrugs.

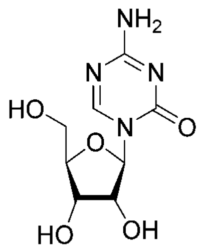

azacitidine
(II)

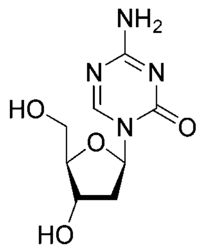

decitabine
(III)

(2) BCL-2 inhibitor

A BCL-2 inhibitor used in the present invention refers to a drug having inhibitory actions against the physiological functions of the BCL-2 in cells, and encompasses a drug which inhibits the physiological functions of the BCL-2 such as a low molecular weight compound, a polypeptide, a protein, a nucleic acid (siRNA, miRNA, aptamer, or the like), and another high molecular weight compound.

BCL-2 is a member of the BCL-2 family that regulates cell death, and negatively regulates apoptosis. BCL-2 is activated in lymphoid B cell tumors such as follicular lymphoma, DLBCL, and CLL, as well as in multiple myeloma and T cell tumors. A BCL-2 inhibitor induces apoptosis in these cancer cells, thereby demonstrating antitumor effects. Thus, even greater antitumor effects can be expected when used alone or in combination with other drugs.

Examples of the BCL-2 inhibitor include venetoclax, navitoclax, obatoclax, obatoclax mesylate, sabutoclax, APG-1252, AZD-0466, APG-2575, ABBV-167, S-65487, and S-55746.

(3) Pharmaceutical composition comprising compound (I)

In the present invention, the compound (I) is a furanone derivative (I) represented by ethyl 5-[(1H-pyrrolo[2,3-b]pyridin-3-yl)methylene]-4-oxo-2-{[4-(2,2,2-trifluoroethyl)piperazinyl]amino}-4,5-dihydrofuran-3-carboxylate:

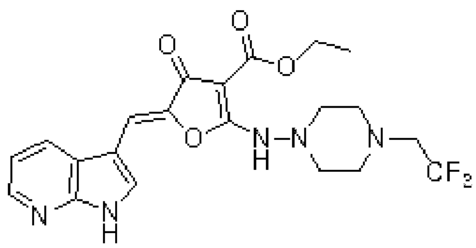

(I)

and pharmaceutically acceptable salts, hydrates, and prodrugs thereof are also encompassed by the compound (I).

The compound (I) is disclosed in WO 2012/133802 pamphlet (Patent Document 1) and Nonpatent Document 5, and known as a compound which selectively and potently inhibits CDC7.

Also, examples of the pharmaceutically acceptable salt of the compound (I) used in the present invention include inorganic acid salts with hydrochloric acid, sulfuric acid, carbonic acid, phosphoric acid, or the like; and organic acid salts with fumaric acid, maleic acid, methanesulfonic acid, p-toluenesulfonic acid, or the like. Further, alkali metal salts with sodium, potassium, or the like; alkaline earth metal salts with magnesium, calcium, or the like; organic amine salts with triethylamine, ethanolamine, or the like; basic amino acid salts with lysine, arginine, ornithine, or the like; ammonium salts; and the like are also encompassed by the present invention.

The compound (I) used in the present invention or a pharmaceutically acceptable salt or hydrate thereof can be prepared in the form of a conventional pharmaceutical formulation (pharmaceutical composition) suitable for oral administration, parenteral administration, or topical administration.

Formulations for oral administration include solid dosage forms such as tablets, granules, powders, and capsules, as well as liquid formulations such as syrups. These formulations can be prepared by conventional methods. Solid dosage forms can be prepared using conventional pharmaceutical carriers such as lactose, starches like corn starch, crystalline cellulose like microcrystalline cellulose, hydroxypropyl cellulose, calcium carboxymethyl cellulose, talc, and magnesium stearate. Capsules can be prepared by encapsulating granules or powders prepared in this manner. Syrups can be prepared by dissolving or suspending the compound or a pharmaceutically acceptable salt or hydrate thereof of the present invention in an aqueous solution comprising sucrose, carboxymethylcellulose, or the like.

Formulations for parenteral administration include injectables such as intravenous infusions. Injectable formulations can also be prepared by conventional methods by appropriately incorporating the compound or a pharmaceutically acceptable salt or hydrate thereof of the present invention into isotonic agents (e.g., mannitol, sodium chloride, glucose, sorbitol, glycerol, xylitol, fructose, maltose, or mannose), stabilizers (e.g., sodium sulfite, or albumin), or preservatives (e.g., benzyl alcohol, or methyl p-hydroxybenzoate).

(4) Combination therapy

In the combination therapy of the present invention, a two-drug combination in which a pharmaceutical composition comprising the compound (I) as an active ingredient is combined with a DNA methyltransferase inhibitor, or a three-drug combination in which a pharmaceutical composition comprising the compound (I) as an active ingredient is combined with a DNA methyltransferase inhibitor and a BCL-2 inhibitor, is administered to a blood cancer patient.

One aspect of the present invention also encompasses a kit comprising a pharmaceutical composition comprising the compound (I) as an active ingredient administered in combination with a DNA methyltransferase inhibitor, wherein the pharmaceutical composition comprising the DNA methyltransferase inhibitor and the compound (I), each as an active ingredient. Said kit may include the instructions for use for both drugs.

One aspect of the present invention is a pharmaceutical composition comprising the compound (I) as an active ingredient administered simultaneously with a DNA methyltransferase inhibitor.

Another aspect of the present invention is a pharmaceutical composition comprising the compound (I) as an active ingredient administered after the administration of a DNA methyltransferase inhibitor.

Another aspect of the present invention is a pharmaceutical composition comprising the compound (I) as an active ingredient administered before the administration of a DNA methyltransferase inhibitor.

One aspect of the present invention also encompasses a kit comprising a pharmaceutical composition comprising the compound (I) as an active ingredient administered in combination with a DNA methyltransferase inhibitor and a BCL-2 inhibitor, wherein the pharmaceutical composition comprises the DNA methyltransferase inhibitor, the BCL-2 inhibitor, and the compound (I), each as an active ingredient. Said kit may include the instructions for use for both drugs.

One aspect of the present invention is a pharmaceutical composition comprising the compound (I) as an active ingredient administered simultaneously with a DNA methyltransferase inhibitor and a BCL-2 inhibitor.

Another aspect of the present invention is a pharmaceutical composition comprising the compound (I) as an active ingredient administered after the administration of a DNA methyltransferase inhibitor and a BCL-2 inhibitor.

Another aspect of the present invention is a pharmaceutical composition comprising the compound (I) as an active ingredient administered before the administration of a DNA methyltransferase inhibitor and a BCL-2 inhibitor.

Azacitidine, decitabine, and venetoclax are already marketed and used for treating blood cancers. A skilled person can appropriately determine the dose of azacitidine or decitabine in the combination therapy of the present invention, based on the doses in which each of them is administered as a single agent, taking into account of the type of blood cancer, the patient's weight, age, severity of the disease, and the like.

Furthermore, a skilled person can appropriately determine the doses of a DNA methyltransferase inhibitor such as azacitidine and a BCL-2 inhibitor such as venetoclax in the three-drug combination therapy of the present invention, based on the doses in which they are administered in a combination therapy, taking into account of the type of blood cancer,

the patient's weight, age, severity of the disease, and the like.

The dose of the compound (I) used in the present invention may be adjusted according to the severity of the disease, the patient's age and body weight, the dosage form, and the like, and it is usually within the range of 10 mg to 400 mg per day for adults. Also, it may be administered orally or parenterally, either as a single dose or divided into two or three doses.

EXAMPLES

Test Example 1. Cell growth suppression test in monotherapy

(Cell culture)

A human acute monocytic leukemia cell line (THP-1), a human acute myeloid leukemia cell line (MV-4-11), a human acute monocytic leukemia cell line (MOLM-14), and a human erythroleukemia cell line (TF-1) were cultured in a 5% $CO_2$ incubator using a RPMI1640 medium (Roswell Park Memorial Institute medium, nacalai tesque) comprising 10% Fetal bovine serum (Thermo Scientific, SIGMA-ALDRICH, or Cytiva) and 1% penicillin/streptomycin (nacalai tesque).

(Cell growth suppression test)

THP-1 cells and MV-4-11 cells at $2\times10^4$ cells/well, and MOLM-14 cells and TF-1 cells at $5\times10^3$ cells/well were each seeded to a 96 well plate (cell plate), and thereto was added a test compound diluted with the medium so that the final concentration thereof would be 3 nM to 30 μM (final DMSO concentration: 0.3%). After the resulting mixture was cultured for 72 hours, a resazurin reagent (nacalai tesque) was added thereto. After 3 hours, a fluorescence at an excitation wavelength of 560 nm and a fluorescence wavelength of 590 nm was measured, and each $IC_{50}$ value of the inhibitory activity was calculated by a criterion in which a well without the compound and without the cells was set to be 100%, and a well without the compound and with cells was set to be 0%. The results are shown in Table 1.

[Table 1]

| | Cell growth suppression activity $IC_{50}$ (μM) | | | |
|---|---|---|---|---|
| | THP-1 | MV-4-11 | MOLM-14 | TF-1 |
| Azacitidine | 7.39 | 1.20 | 2.85 | 1.92 |
| Decitabine | >30 | 0.54 | 0.16 | 0.12 |
| Compound (I) | 4.07 | 0.19 | 0.16 | 0.34 |

Example 1 Dose-response test of DNA methyltransferase inhibitor in the presence of compound (I)

Changes in the $IC_{50}$ value of a DNA methyltransferase inhibitor (azacitidine) in the presence of the compound (I) were investigated.

In view of the results of the monotherapy of the compound (I), a concentration of the compound (I) for one dose was selected, a DMSO-added group was used as the control, and the DNA methyltransferase inhibitor was added to the cell plate so that the concentrations would be ranging from 3 nM to 30 μM (final DMSO concentration: 0.4%). The $IC_{50}$ value in the presence of the compound (I) was determined in a similar manner to the Test Example 1.

Changes in $IC_{50}$ value of azacitidine in the presence of the compound (I) when THP-1 cells were used are shown in Figure 1 and Table 2.

In this test, as shown in Figure 1 and Table 2, in a human acute monocytic leukemia cell line, i.e., in THP-1 cells, $IC_{50}$ values of azacitidine were decreased in the presence of the compound (I).

[Table 2]

| Concentration of compound (I) (μM) | THP-1 cell growth suppression activity azacitidine $IC_{50}$ (μM) |
|---|---|
| 0 | 6. 93 |
| 3 | 2.03 |

The results of Example 1 demonstrate that the combination of the compound (I) and a DNA methyltransferase

inhibitor of the present invention exhibits combined effects against blood cancer cells.

Example 2. Assessment of DNA damage and apoptosis induction

Effects of the combination of a DNA methyltransferase inhibitor and the compound (I) were investigated as changes in the expression levels of a DNA damage indicator, γH2AX, and an apoptosis indicator, cleaved caspase 3.

THP-1 cells or MV-4-11 cells at $2\times10^5$ cells/well were seeded to a 12 well plate, and cultured in a $CO_2$ incubator overnight. The day after the seeding, each of the compound (I) and azacitidine or decitabine was added to the cells, and the resulting mixture was additionally incubated for 24 hours. After 24 hours from the drug addition, the cells were dissolved using a RIPA buffer, and proteins were harvested. Expression of each protein was determined by Western blotting using an anti-γH2AX antibody and an anti-cleaved caspase 3 antibody (Cell Signaling Technology, Inc.).

As shown in Figure 2A and Figure 2B, the γH2AX induction and caspase 3 cleavage by the DNA methyltransferase inhibitor were promoted in the presence of the compound (I). This demonstrates that the combination of the compound (I) and a DNA methyltransferase inhibitor promotes DNA damage and cell death in blood cancers.

Example 3. Assessment of apoptosis and cell death induction

The apoptosis induction by a combination of a DNA methyltransferase inhibitor and the compound (I) was investigated.

THP-1 cells at $1\times10^5$ cells/well were seeded to a 24 well plate, and each of the compound (I) and azacitidine was added to the cells. The resulting mixture was cultured in a $CO_2$ incubator for 48 hours, and the cells were harvested. The resulting cell suspension was mixed with the same amount of Guava Nexin REAGENT (Millipore), and analyzed by SA3800 flow cytometer (Sony Corporation).

As shown in Figure 3, compared to azacitidine monotherapy, the group treated in combination with the compound (I) demonstrated a significantly increased proportion of dead cells due to apoptosis of annexin V-positive and 7-AAD-positive, which demonstrates potent combined effects between the two drugs.

Example 4. Assessment of drug combination effects of DNA methyltransferase inhibitor and compound (I) by isobologram method

The isobologram method is a method for assessing whether the combined effects of two drugs used together are additive, synergistic, or antagonistic (Chou Cancer Res. 70(2): 440-6(2010)). Using this isobologram method, the combined effects of a DNA methyltransferase inhibitor and the compound (I) on the growth inhibition of blood cancer cells were analyzed.

Based on the results of cell growth suppression test for each cell line in the monotherapy and the concentration corresponding to each $IC_{50}$ value in the monotherapy of the compound (I) or a DNA methyltransferase inhibitor, a DMSO solution at a concentration of 10,000 times of the $IC_{50}$ value was prepared. The two-drug mixtures were prepared at six ratios of 1:0, 5:1, 3:1, 1:1, 1:5, and 0:1. These mixtures were added to cell plates for each cell line to achieve final concentrations ranging from 0.003 to 30 time(s) of the $IC_{50}$ value (final DMSO concentration: 0.3%). After 72 hours of culture, a resazurin reagent was added thereto. After 3 hours, a fluorescence at an excitation wavelength of 560 nm and a fluorescence wavelength of 590 nm was measured to determine the $IC_{50}$ value for each mixture ratio.

Based on the $IC_{50}$ values of the monotherapy and the two-drug mixture ratios, the concentrations of the compound (I) and the DNA methyltransferase inhibitor required to achieve 50% growth inhibitory action were calculated. The results are plotted in Figure 4, with the vertical axis representing the concentration of the DNA methyltransferase inhibitor and the horizontal axis representing the concentration of the compound (I).

(Assessment by isobologram)

For a drug A and a drug B, the doses at which a certain effect is exhibited in monotherapy are designated $D_A$ and $D_B$, respectively. When the dose-response curves of both drugs are parallel, if the dose at which the combined effect of both drugs is the same as when they are administered in the monotherapy is on the straight line $D_AD_B$, it is determined to be an additive effect; if it is below and to the left of the straight line $D_AD_B$, it is determined to be a synergistic effect; and if it is above and to the right of the straight line $D_AD_B$, it is determined to be an antagonistic effect.

In this test, as shown in Figure 4A, Figure 4B, Figure 4C, Figure 4D, Figure 4E, Figure 4F, and Figure 4G, the concentration of each mixture ratio required to exhibit 50% growth inhibitory action is below and to the left of the straight line connecting the $IC_{50}$ values in the monotherapy. Therefore, the results of Example 4 demonstrate that the combination of the compound (I) and a DNA methyltransferase inhibitor of the present invention exhibits potent synergistic effects.

Example 5. Assessment of drug combination effects of DNA methyltransferase inhibitor and compound (I) by Median-effect analysis

Median-effect analysis is a theory proposed by Chou and Talalay, which is a method for assessing whether the combined effects of two drugs used together are additive, synergistic, or antagonistic (Chou Cancer Res., 70(2): 440-6(2010)). When the drug concentration is D, 50% inhibitory concentration (median-effect dose) is *Dm,* suppressed cell rate is *Fa* (fraction affected), non-suppressed cell rate is *Fu* (fraction unaffected), and m is a coefficient, then the following equation is satisfied.

$$D=Dm\,(Fa/Fu)^{1/m}$$

When the sites of action for effects of two drugs are exclusive, the combination index CI, which a quantitative measure of combined effects, is given by the following equation: where the concentration of drug A during combination therapy is denoted as $(D_{A+B})_A$, the x% inhibitory concentration of drug A alone is denoted as $(Dx)_A$, the concentration of drug B during combination therapy is denoted as $(D_{A+B})_B$, and the x% inhibitory concentration of drug B alone is denoted as $(Dx)_B$.

$$CI=(D_{A+B})_A/(Dx)_A+(D_{A+B})_B/(Dx)_B$$

CI is expressed as a function of *Fa* as described above. Plotting *Fa* on the horizontal axis and CI on the vertical axis yields a correlation figure called an *Fa-CI plot.* A CI value less than 1 indicates synergistic effects, 1 indicates additive effects, and greater than 1 indicates antagonistic effects. Using the experimental data from the isobologram in Example 4, the combination index CI was calculated for the DNA methyltransferase inhibitor and the compound (I) in blood cancer cells when *Fa* = 0.5.

[Table 3]

| Cell line | Azacitidine + Compound (I) Combination index CI |
|---|---|
| THP-1 | 0.66 |
| MV-4-11 | 0.84 |
| MOLM-14 | 0.81 |
| TF-1 | 0.81 |

[Table 4]

| Cell line | Decitabine + Compound (I) Combination index CI |
|---|---|
| MV-4-11 | 0.36 |
| MOLM-14 | 0.51 |
| TF-1 | 0.78 |

As shown in Table 3 and Table 4, the CI values were less than 1, indicating potent synergistic effects when the DNA methyltransferase inhibitor was used in combination with the compound (I).

Example 6. Assessment of three-drug combination effects when compound (I) is combined with two-drugs of DNA methyltransferase inhibitor and BCL-2 inhibitor

Using a similar method to the Median-effect analysis of Chou-Talalay in Example 5, CI values in three-drug combination were calculated.

When the concentration of drug A during combination therapy is denoted as $(D_{A+B+C})_A$, the x% inhibitory concentration of drug A alone is denoted as $(Dx)_A$, the concentration of drug B during combination therapy is denoted as $(D_{A+B+C})_B$, the x% inhibitory concentration of drug B alone is denoted as $(Dx)_B$, the concentration of drug C during combination therapy is denoted as $(D_{A+B+C})_C$, and the x% inhibitory concentration of drug C alone is denoted as $(Dx)_C$, CI is given by the following equation.

$$CI = (D_{A+B+C})_A / (Dx)_A + (D_{A+B+C})_B / (Dx)_B + (D_{A+B+C})_C / (Dx)_C$$

The combination index CI was calculated for the DNA methyltransferase inhibitor, the BCL-2 inhibitor, and the compound (I) in blood cancer cells when *Fa* = 0.5.

[Table 5]

| Cell line | Azacitidine + Venetoclax Combination index CI | Azacitidine + Venetoclax + Compound (I) Combination index CI |
|---|---|---|
| MV-4-11 | 0.70 | 0.52 |
| MOLM-14 | 0.75 | 0.63 |

As shown in Figure 5 and Table 5, CI values were smaller in the three-drug combination when the compound (I) was used in combination with the DNA methyltransferase inhibitor and the BCL-2 inhibitor compared to the two-drug combination of the DNA methyltransferase inhibitor and the BCL-2 inhibitor, indicating that the three-drug combination of the compound (I) with the DNA methyltransferase inhibitor and the BCL-2 inhibitor exhibits more potent synergistic effects.

Example 7. Antitumor effects in the combination therapy of compound (I) and DNA methyltransferase inhibitor

The drug combination effects of the compound (I) and a DNA methyltransferase inhibitor (azacitidine or decitabine) were investigated using a nude mouse subcutaneous transplantation model of MV-4-11 cell line.

(Preparation of cancer-bearing model)

MV-4-11 cells cultured in a similar manner to Test Example 1 were adjusted to a cell density of $2.5 \times 10^7$ cells/mL in HBSS (nacalai tesque) containing Matrigel (50%, BD) to prepare a cell preparation solution for transplantation. A 0.1 mL aliquot of this cell preparation solution for transplantation was injected subcutaneously into the dorsal region of a BALB/c Slc-nu/nu mouse (female, 5 weeks old, Japan SLC, Inc.). Grouping was made such that the mean tumor volume (see calculation formula below) of cancer-bearing mice was similar on day 20 after cancer cell transplantation (n=8).

(Preparation of sample solution for administration of test substance)

A dihydrochloride of the compound (I) was dissolved in a 0.1 M HCl / 0.5% methylcellulose solution (Solvent A) to give 6 mg/mL in terms of free base, to prepare a sample solution for administration of the compound (I).
Azacitidine was dissolved in a 5% (v/v) DMSO / 30% (w/v) PEG 300 aqueous solution (Solvent B) to give a concentration of 0.5 mg/mL, to prepare a sample solution for administration of azacitidine.
Decitabine was dissolved in distilled water (Solvent C) to give a concentration of 0.025 mg/mL, to prepare a sample solution for administration of decitabine.

(Antitumor effect test)

Each mouse (n=7-8) transplanted with cancer cells received each test substance of azacitidine (Table 6) or decitabine (Table 7) according to the respective administration schedule. The test substance dose, calculated from each mouse's body weight on the day of administration, was orally (PO) or intraperitoneally (IP) administered either twice daily (BID, at least 5-hour intervals) or once daily (QD). The tumor volume for each mouse was calculated using the following equation to assess the antitumor effects.

Tumor volume = long diameter × short diameter × short diameter ×0.5

[Table 6]

| Test group | Test substance | Administration route | Dose (mg/kg) | Administration schedule |
|---|---|---|---|---|
| G1 | Solvent B<br>Solvent A | IP<br>PO | -<br>- | QD, D1-D19 (off: D6, D7, D13, D14)<br>BID, D1-D19 (off: D6, D7, D13, D14) |

(continued)

| Test group | Test substance | Administration route | Dose (mg/kg) | Administration schedule |
|---|---|---|---|---|
| G2 | Azacitidine<br>Solvent A | IP<br>PO | 2.5<br>- | QD, D1-D19 (off: D6, D7, D13, D14)<br>BID, D1-D19 (off: D6, D7, D13, D14) |
| G3 | Solvent B<br>Compound (I) | IP<br>PO | -<br>60 | QD, D1-D19 (off: D6, D7, D13, D14)<br>BID, D1-D19 (off: D6, D7, D13, D14) |
| G4 | Azacitidine<br>Compound (I) | IP<br>PO | 2.5<br>60 | QD, D1-D19 (off: D6, D7, D13, D14)<br>BID, D1-D19 (off: D6, D7, D13, D14) |

The term "off" represents a drug holiday.

[Table 7]

| Test group | Test substance | Administration route | Dose (mg/kg) | Administration schedule |
|---|---|---|---|---|
| G5 | Solvent C<br>Solvent A | IP<br>PO | -<br>- | QD, D1-D19 (off: D6, D7, D13, D14)<br>BID, D1-D19 (off: D6, D7, D13, D14) |
| G6 | Decitabine<br>Solvent A | IP<br>PO | 0.25<br>- | QD, D1-D19 (off: D6, D7, D13, D14)<br>BID, D1-D19 (off: D6, D7, D13, D14) |
| G7 | Solvent C<br>Compound (I) | IP<br>PO | -<br>60 | QD, D1-D19 (off: D6, D7, D13, D14)<br>BID, D1-D19 (off: D6, D7, D13, D14) |
| G8 | Decitabine<br>Compound (I) | IP<br>PO | 0.25<br>60 | QD, D1-D19 (off: D6, D7, D13, D14)<br>BID, D1-D19 (off: D6, D7, D13, D14) |

The term "off" represents a drug holiday.

In this test, as shown in Figure 6A and Figure 6B, tumor growth was most effectively suppressed in the group treated with the compound (I) in combination with a DNA methyltransferase inhibitor (azacitidine or decitabine). The results of Example 7 demonstrate that the combination of the compound (I) with a DNA methyltransferase inhibitor of the present invention exhibits potent synergistic effects even in vivo.

Example 8. Antitumor effects in three-drug combination in which compound (I) is combined with two-drug of DNA methyltransferase inhibitor and BCL-2 inhibitor

Effects of three-drug combination were investigated using a nude mouse subcutaneous transplantation model of MV-4-11 cell line in a similar manner to Example 7.

(Preparation of sample solution for administration of test substance)

A dihydrochloride of the compound (I) was dissolved in a 0.1 M HCl / 0.5% methylcellulose solution (Solvent A) to give 3 mg/mL in terms of free base, to prepare a sample solution for administration of the compound (I).

Azacitidine was dissolved in a 5% (v/v) DMSO / 30% (w/v) PEG 300 aqueous solution (Solvent B) to give concentrations of 0.25 mg/mL and 0.125 mg/mL, to prepare sample solutions for administration of azacitidine.

Decitabine was dissolved in distilled water (Solvent C) to give a concentration of 0.025 mg/mL, to prepare a sample solution for administration of decitabine.

Venetoclax was dissolved in a 5% (v/v) DMSO / 50% (w/v) PEG 300 / 5% (w/v) Tween80 aqueous solution (Solvent D) to give concentrations of 0.5 mg/mL and 0.25 mg/mL, to prepare sample solutions for administration of venetoclax.

(Antitumor effect test)

Grouping was made at Day 19 from the transplantation of cancer cells. Each mouse (n=6-8) transplanted with cancer cells received each test substance according to the respective administration schedule show in Table 8 or Table 9. The test substance dose, calculated from each mouse's body weight on the day of administration, was orally (PO) or intraperitoneally (IP) administered either twice daily (BID, at least 5-hour intervals) or once daily (QD), and the antitumor

effects were assessed.

[Table 8]

| Test group | Test substance | Administration route | Dose (mg/kg) | Administration schedule |
|---|---|---|---|---|
| G1 | Solvent B<br>Solvent D<br>Solvent A | IP<br>PO<br>PO | -<br>-<br>- | QD, D1-D19 (off: D6, D7, D13, D14)<br>QD, D1-D19 (off: D6, D7, D13, D14)<br>BID, D1-D19 |
| G2 | Solvent B<br>Solvent D<br>Compound (I) | IP<br>PO<br>PO | -<br>-<br>30 | QD, D1-D19 (off: D6, D7, D13, D14)<br>QD, D1-D19 (off: D6, D7, D13, D14)<br>BID, D1-D19 |
| G3 | Azacitidine<br>Venetoclax<br>Solvent A | IP<br>PO<br>PO | 0.625<br>5<br>- | QD, D1-D19 (off: D6, D7, D13, D14)<br>QD, D1-D19 (off: D6, D7, D13, D14)<br>BID, D1-D19 |
| G4 | Azacitidine<br>Venetoclax<br>Compound (I) | IP<br>PO<br>PO | 0.625<br>5<br>30 | QD, D1-D19 (off: D6, D7, D13, D14)<br>QD, D1-D19 (off: D6, D7, D13, D14)<br>BID, D1-D19 |

The term "off" represents a drug holiday.

[Table 9]

| Test group | Test substance | Administrati on route | Dose (mg/kg) | Administration schedule |
|---|---|---|---|---|
| G5 | Solvent C<br>Solvent D<br>Solvent A | IP<br>PO<br>PO | -<br>-<br>- | QD, D1-D19 (off: D6, D7, D13, D14)<br>QD, D1-D19 (off: D6, D7, D13, D14)<br>BID, D1-D19 |
| G6 | Solvent C<br>Solvent D<br>Compound (I) | IP<br>PO<br>PO | -<br>-<br>30 | QD, D1-D19 (off: D6, D7, D13, D14)<br>QD, D1-D19 (off: D6, D7, D13, D14)<br>BID, D1-D19 |
| G7 | Decitabine<br>Venetoclax<br>Solvent A | IP<br>PO<br>PO | 0.25<br>5<br>- | QD, D1-D19 (off: D6, D7, D13, D14)<br>QD, D1-D19 (off: D6, D7, D13, D14)<br>BID, D1-D19 |
| G8 | Decitabine<br>Venetoclax<br>Compound (I) | IP<br>PO<br>PO | 0.25<br>5<br>30 | QD, D1-D19 (off: D6, D7, D13, D14)<br>QD, D1-D19 (off: D6, D7, D13, D14)<br>BID, D1-D19 |

The term "off" represents a drug holiday.

In this test, as shown in Figure 7A and Figure 7B, tumor growth was most effectively suppressed in the three-drug combined group of the compound (I) with a DNA methyltransferase inhibitor and a BCL-2 inhibitor. The results of Example 8 demonstrate that the three-drug combination of the compound (I) with a DNA methyltransferase inhibitor and a BCL-2 inhibitor of the present invention exhibits potent synergistic effects even in vivo.

INDUSTRIAL APPLICABILITY

According to the present invention, more effective methods for treating blood cancers, and combinations of pharmaceutical compositions or kits to carry out the methods are provided by combining two drugs of the compound (I) and a DNA methyltransferase inhibitor, or combining three drugs of the compound (I), a DNA methyltransferase inhibitor, and a BCL-2 inhibitor.

## Claims

**1.** A pharmaceutical composition comprising a compound (I) :

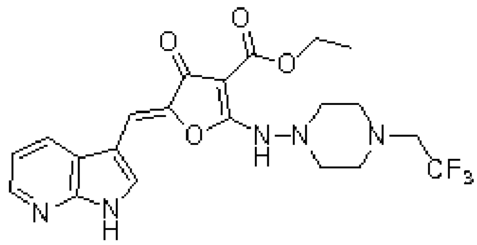

(I)

or a pharmaceutically acceptable salt or hydrate thereof as an active ingredient, which is administered to a patient in combination with a DNA methyltransferase inhibitor for use in the treatment of a blood cancer.

**2.** The pharmaceutical composition according to claim 1, which is administered to the patient further in combination with a BCL-2 inhibitor in addition to the DNA methyltransferase inhibitor for use in the treatment of the blood cancer.

**3.** The pharmaceutical composition according to claim 1 or 2, wherein the blood cancer is myelodysplastic syndrome (MDS) and/or acute myeloid leukemia (AML).

**4.** The pharmaceutical composition according to claim 1 or 2, wherein the blood cancer is myelodysplastic syndrome (MDS).

**5.** The pharmaceutical composition according to claim 1 or 2, wherein the blood cancer is acute myeloid leukemia (AML).

**6.** The pharmaceutical composition according to claim 1 or 2, wherein the DNA methyltransferase inhibitor is a pharmaceutical composition comprising azacitidine or a pharmaceutically acceptable salt or hydrate thereof, or decitabine or a pharmaceutically acceptable salt or hydrate thereof, as an active ingredient.

**7.** The pharmaceutical composition according to claim 6, wherein the DNA methyltransferase inhibitor is a pharmaceutical composition comprising azacitidine or a pharmaceutically acceptable salt or hydrate thereof as an active ingredient.

**8.** The pharmaceutical composition according to claim 6, wherein the DNA methyltransferase inhibitor is a pharmaceutical composition comprising decitabine or a pharmaceutically acceptable salt or hydrate thereof as an active ingredient.

**9.** The pharmaceutical composition according to claim 7, wherein the pharmaceutical composition comprising azacitidine or a pharmaceutically acceptable salt or hydrate thereof as an active ingredient, and the pharmaceutical composition comprising the compound (I) or a pharmaceutically acceptable salt or hydrate thereof as an active ingredient are administered both simultaneously, or separately and sequentially.

**10.** The pharmaceutical composition according to claim 8, wherein the pharmaceutical composition comprising decitabine or a pharmaceutically acceptable salt or hydrate thereof as an active ingredient, and the pharmaceutical composition comprising the compound (I) or a pharmaceutically acceptable salt or hydrate thereof as an active ingredient are administered both simultaneously, or separately and sequentially.

**11.** The pharmaceutical composition according to claim 2, wherein the BCL-2 inhibitor is a pharmaceutical composition comprising venetoclax or a pharmaceutically acceptable salt or hydrate thereof as an active ingredient.

**12.** The pharmaceutical composition according to any one of claims 9 to 11, wherein the blood cancer is myelodysplastic syndrome (MDS) and/or acute myeloid leukemia (AML).

**13.** The pharmaceutical composition according to claim 12, wherein the blood cancer is myelodysplastic syndrome (MDS).

**14.** The pharmaceutical composition according to claim 12, wherein the blood cancer is acute myeloid leukemia (AML).

**15.** A kit comprising a pharmaceutical composition comprising the DNA methyltransferase inhibitor and the compound (I) or a pharmaceutically acceptable salt or hydrate thereof, each as an active ingredient, for use in the combined administration as defined in claim 1.

**16.** A kit comprising a pharmaceutical composition comprising the DNA methyltransferase inhibitor, the BCL-2 inhibitor, and the compound (I) or a pharmaceutically acceptable salt or hydrate thereof, each as an active ingredient, for use in the combined administration as defined in claim 2.

**17.** A method for treating a blood cancer, the method comprising administering a pharmaceutical composition comprising a compound (I) or a pharmaceutically acceptable salt or hydrate thereof as an active ingredient to a patient, in combination with a DNA methyltransferase inhibitor.

**18.** A method for treating a blood cancer, the method comprising administering a pharmaceutical composition comprising a compound (I) or a pharmaceutically acceptable salt or hydrate thereof as an active ingredient to a patient, in combination with a DNA methyltransferase inhibitor and a BCL-2 inhibitor.

**19.** The method according to claim 17 or 18, wherein the blood cancer is myelodysplastic syndrome (MDS) and/or acute myeloid leukemia (AML).

**20.** The method according to claim 17 or 18, wherein the DNA methyltransferase inhibitor is a pharmaceutical composition comprising azacitidine or a pharmaceutically acceptable salt or hydrate thereof; or decitabine or a pharmaceutically acceptable salt or hydrate thereof, each as an active ingredient.

**21.** The method according to claim 18, wherein the BCL-2 inhibitor is a pharmaceutical composition comprising venetoclax or a pharmaceutically acceptable salt or hydrate thereof as an active ingredient.

[Figure 1]

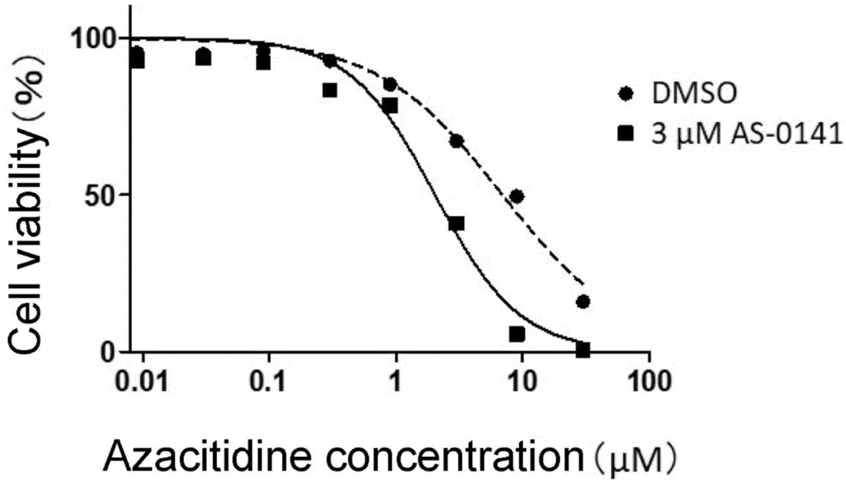


[Figure 2A]

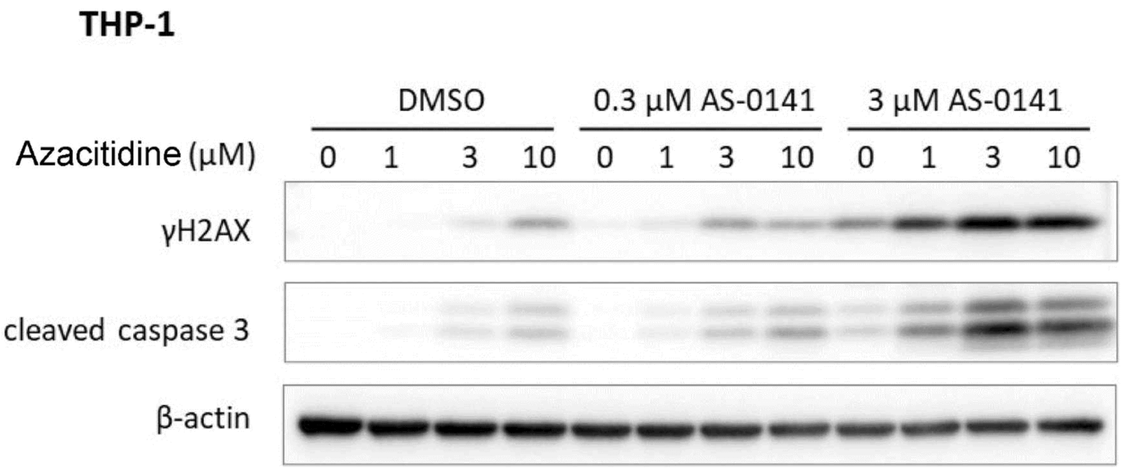

[Figure 2B]

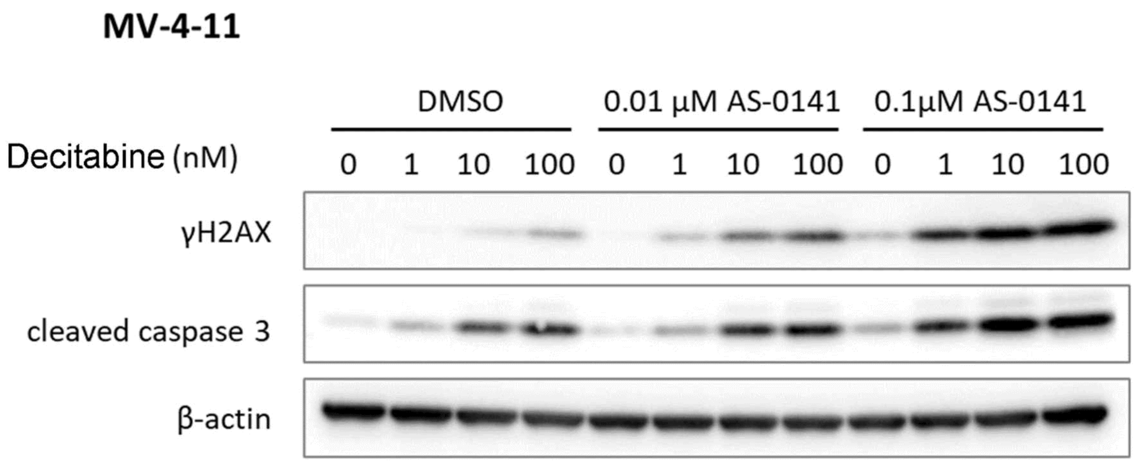
MV-4-11
DMSO
0.01 µM AS-0141
0.1µM AS-0141
Decitabine (nM)
0 1 10 100 0 1 10 100 0 1 10 100
γH2AX
cleaved caspase 3
β-actin

[Figure 3]

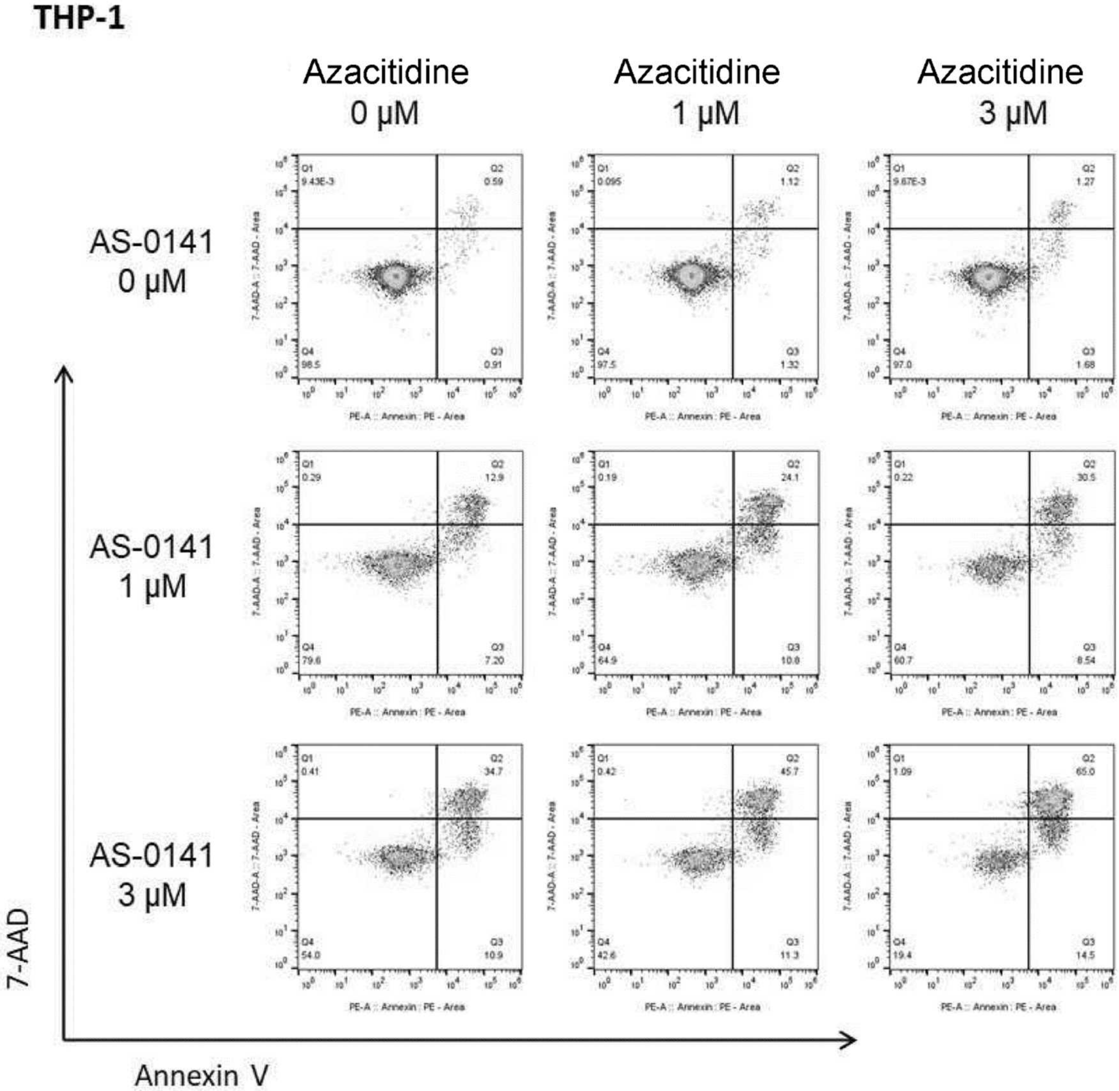
THP-1
Azacitidine 0 μM
Azacitidine 1 μM
Azacitidine 3 μM
AS-0141 0 μM
AS-0141 1 μM
AS-0141 3 μM
7-AAD
Annexin V
Q1 9.43E-3
Q2 0.59
Q4 98.5
Q3 0.91
Q1 0.095
Q2 1.12
Q4 97.5
Q3 1.32
Q1 9.67E-3
Q2 1.27
Q4 97.0
Q3 1.68
Q1 0.29
Q2 12.9
Q4 79.6
Q3 7.20
Q1 0.19
Q2 24.1
Q4 64.9
Q3 10.8
Q1 0.22
Q2 30.5
Q4 60.7
Q3 8.54
Q1 0.41
Q2 34.7
Q4 54.0
Q3 10.9
Q1 0.42
Q2 45.7
Q4 42.6
Q3 11.3
Q1 1.09
Q2 65.0
Q4 19.4
Q3 14.5
PE-A :: Annexin : PE - Area
7-AAD-A :: 7-AAD - Area

[Figure 4A]

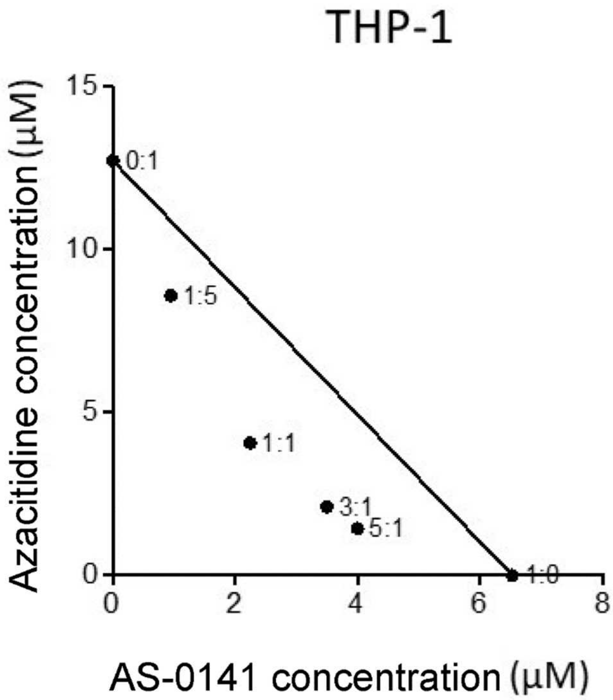


[Figure 4B]

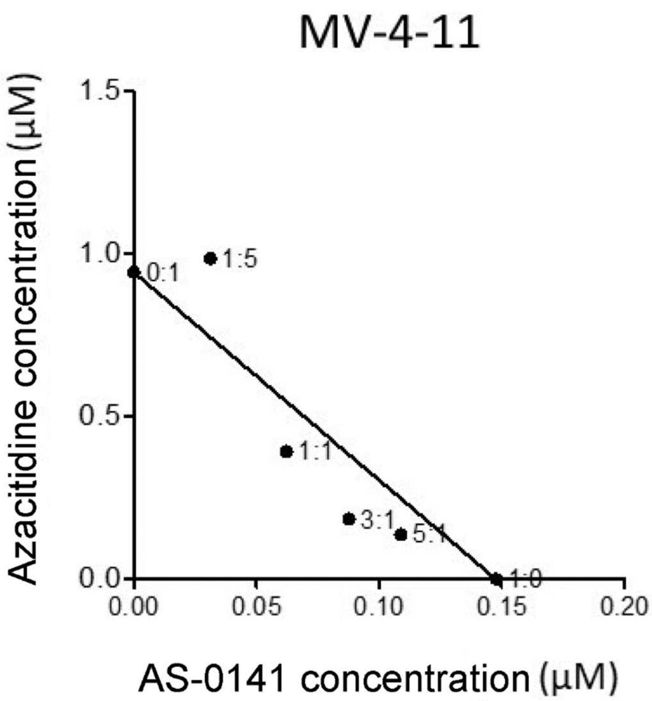

[Figure 4C]

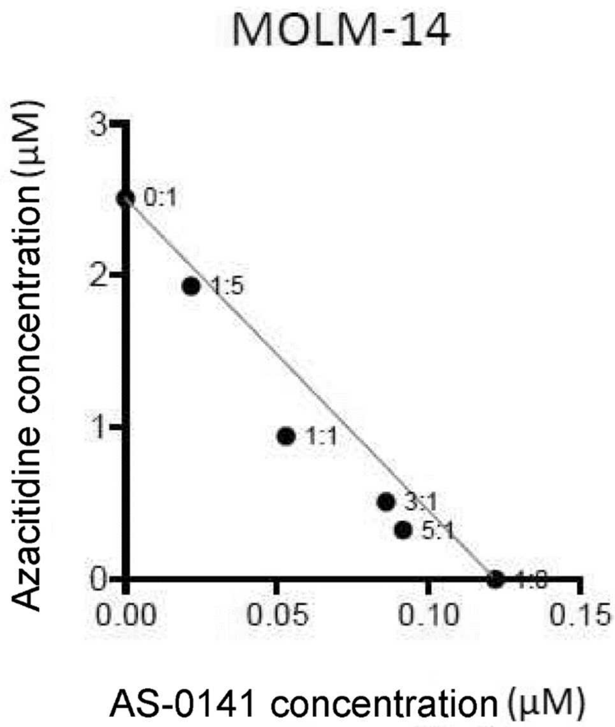


[Figure 4D]

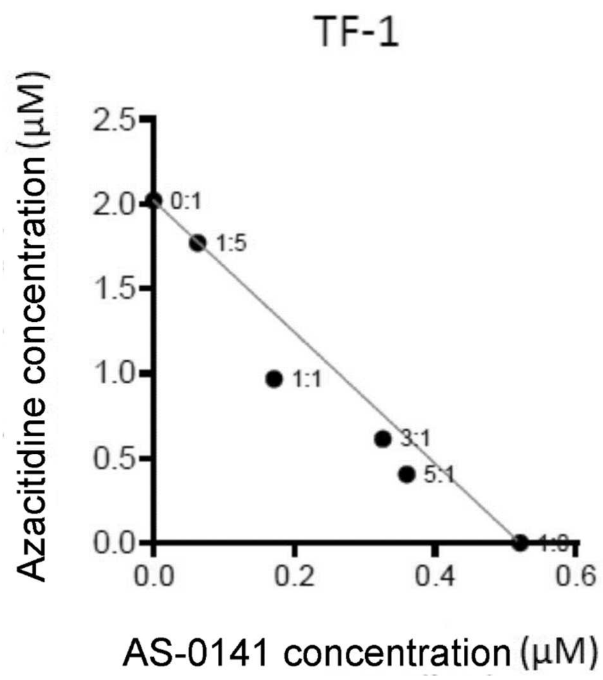

[Figure 4E]

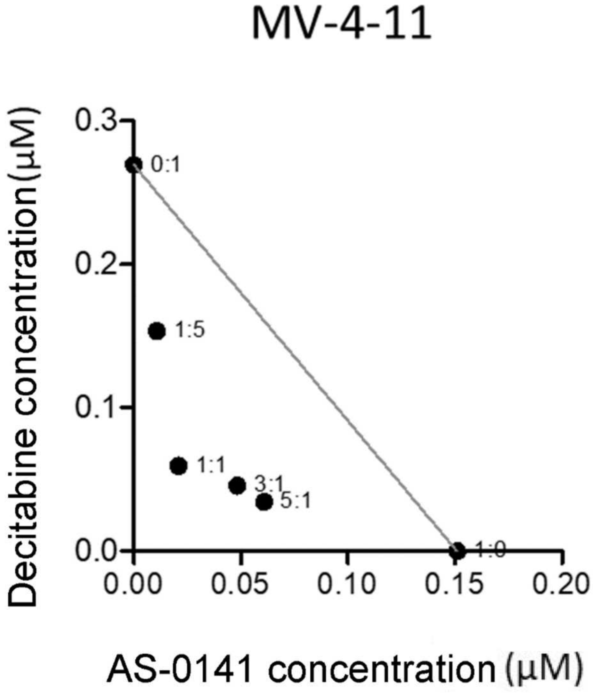


[Figure 4F]

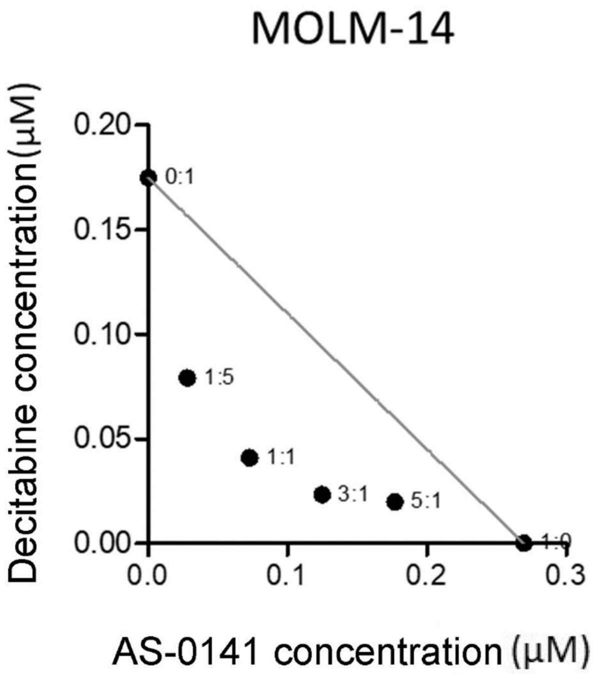

[Figure 4G]

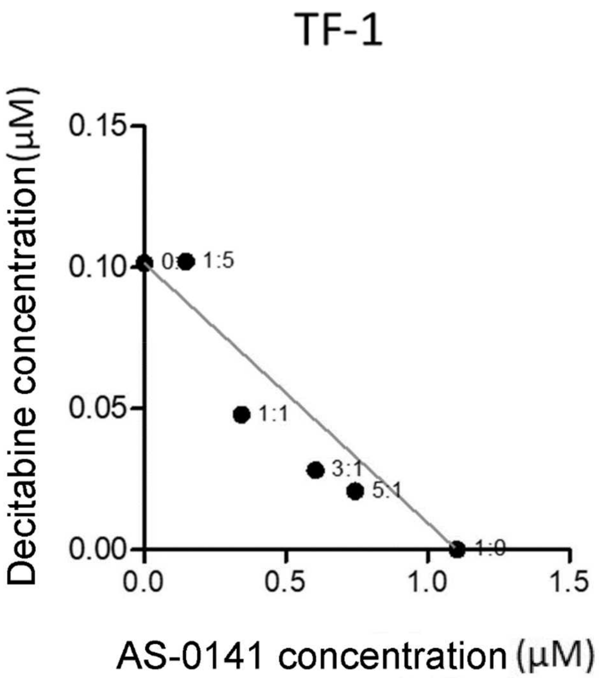


[Figure 5]

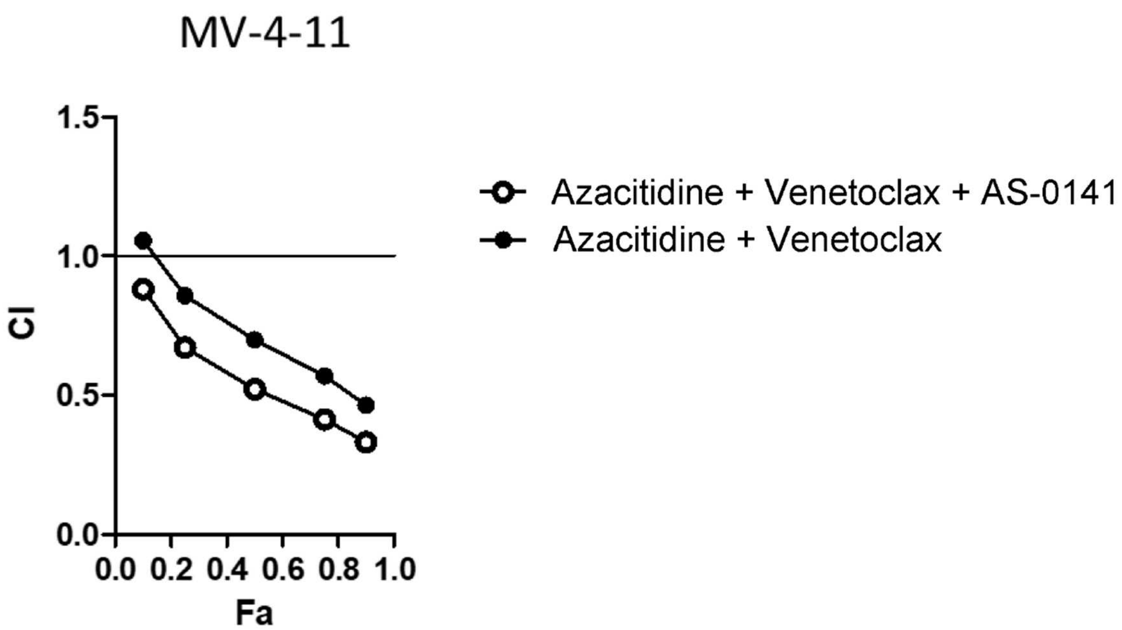

[Figure 6A]

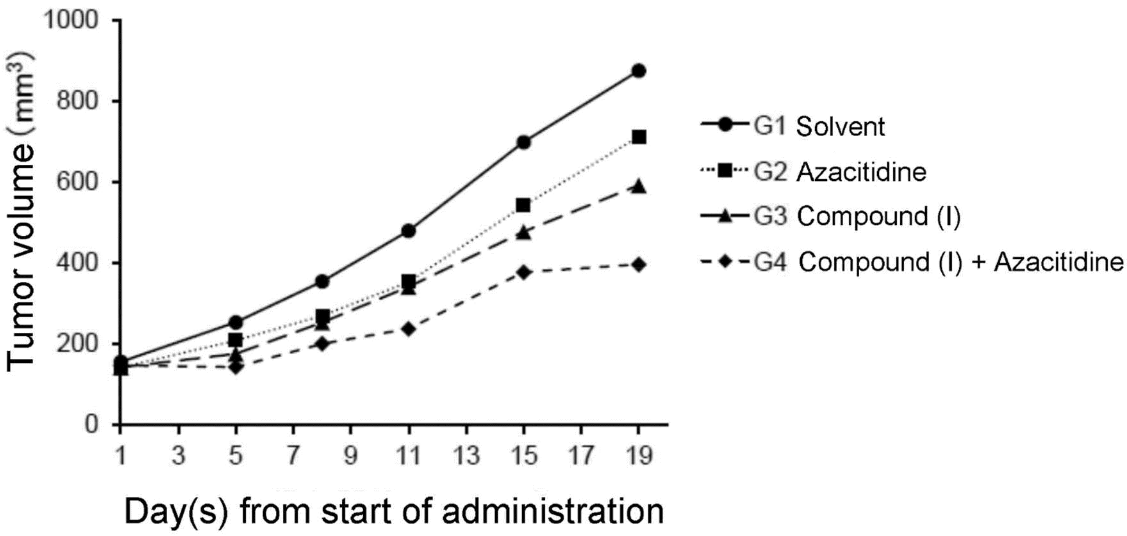


[Figure 6B]

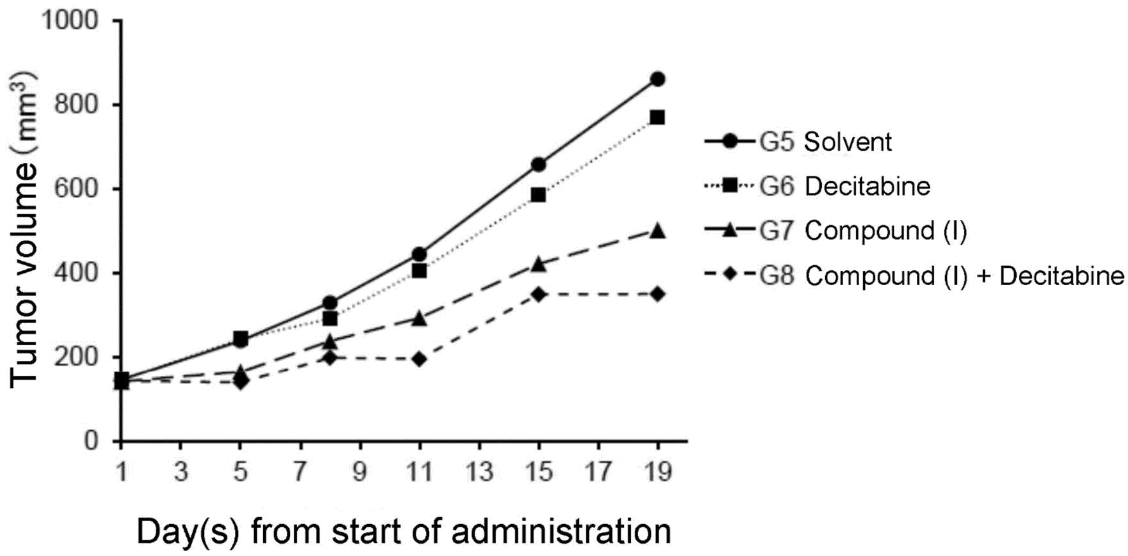

[Figure 7A]

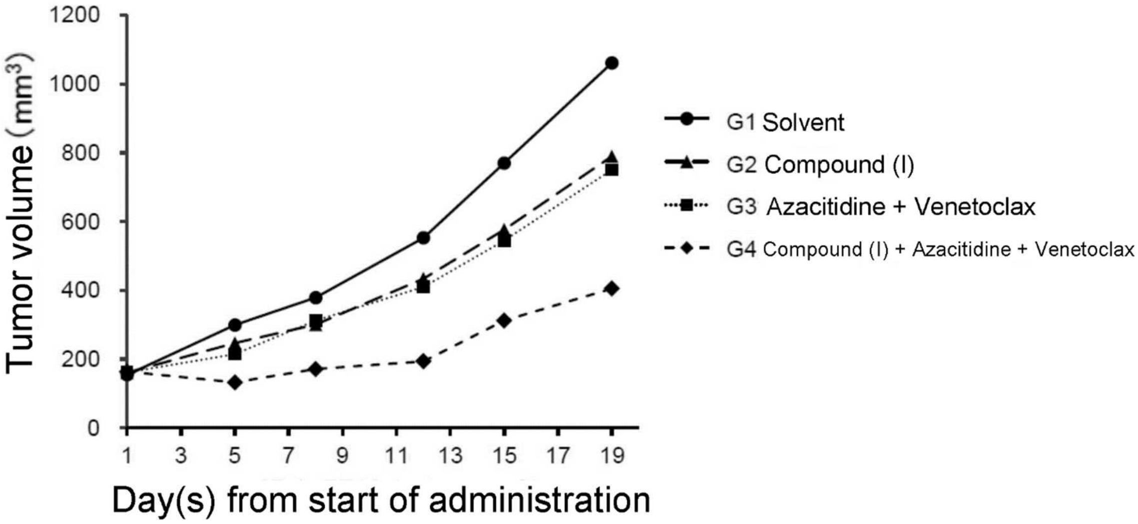


[Figure 7B]

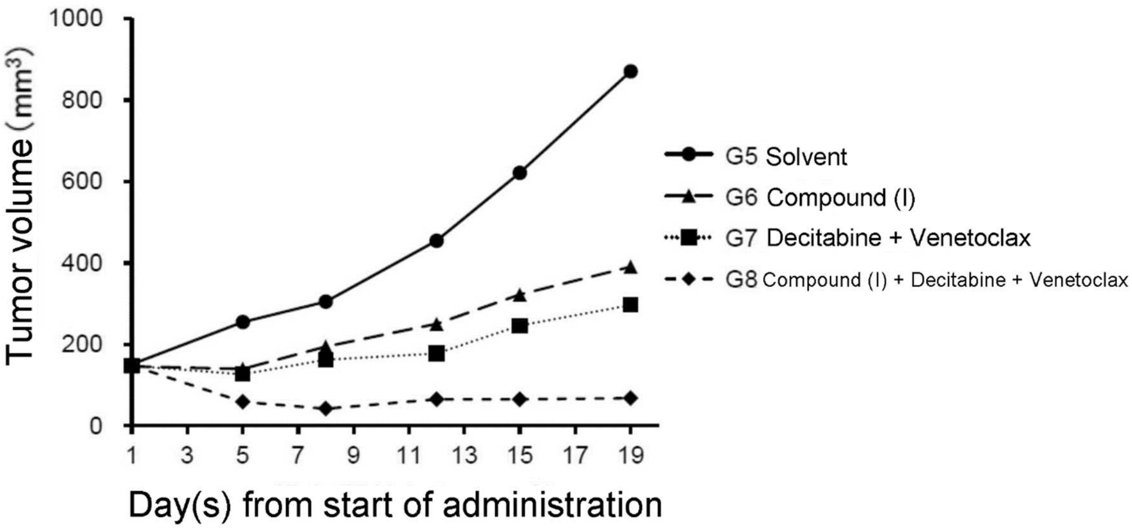

## INTERNATIONAL SEARCH REPORT

International application No.
**PCT/JP2024/034412**

### A. CLASSIFICATION OF SUBJECT MATTER

***A61K 31/496***(2006.01)i; ***A61K 31/7052***(2006.01)i; ***A61K 31/706***(2006.01)i; ***A61K 45/00***(2006.01)i; ***A61P 35/00***(2006.01)i; ***A61P 35/02***(2006.01)i; ***A61P 43/00***(2006.01)i
FI: A61K31/496; A61K31/7052; A61K31/706; A61K45/00; A61P35/00; A61P35/02; A61P43/00 111; A61P43/00 121

According to International Patent Classification (IPC) or to both national classification and IPC

### B. FIELDS SEARCHED

Minimum documentation searched (classification system followed by classification symbols)

A61K31/496; A61K31/7052; A61K31/706; A61K45/00; A61P35/00; A61P35/02; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | JP 2022-500479 A (SIERRA ONCOLOGY, INC.) 04 January 2022 (2022-01-04) claims, paragraphs [0042]-[0044], examples 1-3, 9, 15, 25 | 1-21 |
| Y | JAGANNATHAN, Veena et al. CDC7 kinase inhibition by SRA141 induces a potentially novel caspase-dependent tumor cell apoptosis associated with altered DNA replication and cell cycle dynamics. Cancer Research. 2019, vol. 79, no. 13_Supplement, abstract LB-288 particularly, 1st, 2nd paragraphs | 1-21 |
| Y | 山内　高弘, 抗がん性核酸アナログと白血病の薬物療法, 痛風と核酸代謝, 2017, vol. 41, no. 2, pp. 161-169, (YAMAUCHI, Takahiro, Anticancer nucleoside analogs for treating leukemia, Anticancer nucleoside analogs for treating leukemia) pp. 165-167, "Azacitidine/Decitabine" | 1-21 |
| Y | 石川　裕一, 急性骨髄性白血病の初期診断と外来治療, 日本内科学会雑誌, 2022, vol. 111, no. 7, pp. 1344-1350, (ISHIKAWA, Yuichi, Initial Diagnosis and Outpatient Care for Acute Myeloid Leukemia, Nihon Naika Gakkai Zasshi) pp. 1349-1350, 4.2) AML treatment with BCL2 inhibitors | 1-21 |

☑ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 November 2024** | **10 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/034412**

**C. DOCUMENTS CONSIDERED TO BE RELEVANT**

| Category* | Citation of document, with indication, where appropriate, of the relevant passages | Relevant to claim No. |
|---|---|---|
| P, X | ENDO, Hiroko et al. Synergistic effect of the CDC7 inhibitor, monzosertib(AS-0141) with current therapies in AML models. Cancer Research. 22 March 2024, vol. 84, no. 6_Supplement, abstract 5714<br>particularly, see "Results" | 1-21 |

Form PCT/ISA/210 (second sheet) (July 2022)

**INTERNATIONAL SEARCH REPORT**
**Information on patent family members**

International application No.
**PCT/JP2024/034412**

| Patent document cited in search report | Publication date (day/month/year) | Patent family member(s) | Publication date (day/month/year) |
|---|---|---|---|
| JP 2022-500479 A | 04 January 2022 | WO 2019/165473 A1<br>claims 1-75, paragraphs [0085]-[0087], examples 1-3, 9, 15, 25<br>US 2021/0393620 A1<br>EP 3856352 A1<br>CA 3113621 A<br>KR 10-2021-0064252 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012133802 A
- WO 201884266 A
- WO 202068347 A
- WO 2015115355 A


### Non-patent literature cited in the description


- **ASHKENAZI, A. et al.** *Nature Rev. Drug Discov*, 2017, vol. 16, 273-284
- **ESTEY EH.** *Am J Hematol.*, November 2020, vol. 95 (11), 1368-1398
- **SAWA M** ; **MASAI H**. *Drug Des Devel Ther.*, 06 February 2008, vol. 2, 255-264
- **IRIE T et al.** *J Med Chem.*, 14 October 2021, vol. 64 (19), 14153-14164
- *Chou Cancer Res.*, 2010, vol. 70 (2), 440-6
- **CHOU**. *Cancer Res.*, 2010, vol. 70 (2), 440-6